# EUROPEAN PATENT SPECIFICATION

(11) **EP 0 552 393 B1**
(45) Date of publication and mention of the grant of the patent: **28.05.1997**
(21) Application number: 92101074.0
(22) Date of filing: 23.01.1992
(51) Int. Cl.: C12Q 1/68

(54) **A method for identifying BNYV-Virus resistant beta beet plants or beet seeds as well as a RFLP probe for the accomplishment of said method**
Verfahren zur Identifikation von BNYV-Viren-resistenten Zuckerrübenpflanzen bzw. Saatgut und RFLP-Sonde für die Durchführung dieses Verfahrens
Procédé pour identifier plants et graines de betteraves résistants au virus-BNYV et sonde de RFLP pour réaliser ce procédé

(43) Date of publication of application: 28.07.1993
(73) Proprietor: KWS KLEINWANZLEBENER SAATZUCHT Aktiengesellschaft vorm. Rabbethge & Giesecke, 37555 Einbeck (DE); Max-Planck-Gesellschaft zur Förderung der Wissenschaften e.V., D-37073 Göttingen (DE)
(72) Inventor: Mechelke, Wolfgang, Dr., W-3352 Einbeck (DE); Seitzer, Josef Franz, Dr., W-3352 Einbeck (DE); Salamini, Francesco, Prof., W-5000 Köln 30 (DE); Barzen, Ellen, Dr., W-5000 Köln 30 (DE)
(74) Representative: Behrens, Dieter, Dr.-Ing.

(56) References cited:
- WO-A-89/07647
- CHEMICAL ABSTRACTS, vol. 112, no. 13, 26 March 1990, Columbus, Ohio, US; abstract no. 115055V, T. TOD ET AL.: 'Use of restriction fragment length polymorphism to fingerprint beets at the genotype and species level' page 377 ;column 1 ;
- BIOTECHNOLOGY vol. 7, March 1989, NEW YORK US pages 257 - 264; S. D. TANKSLEY ET AL.: 'RFLP mapping in plant breeding: new tools for an old science.'
- Bodenkultur, 39, 1988, pages 251-258

## Description

### Technical field of the invention

This invention concerns the correlation between one or more agronomically important genes coding for resistance against the rhizomania disease and a Restriction Fragment Length Polymorphism (RFLP) by usage of a RFLP probe, that means a DNA probe. More specifically this invention relates to a method for identifying BNYV-Virus resistant beta beet plants or beet seeds and distinguishing them from BNYV-Virus susceptible beta beet plants or beet seeds as well as to a RFLP probe (Restriction Fragment Length Polymorphism probe) for the identification of rhizomania resistant beta beet plants or beet seeds.

### Background of the Invention

The goal of plant breeding is to combine in a single variety various desirable traits.

For a beet variety, for example a sugar beet variety, this goal includes resistance to the rhizomania virus disease, which is a very important character.

The rhizomania disease of sugar beet is caused by the Beet Necrotic Yellow Vein Virus (BNYVV). It is transmitted by the soil-born fungus Polymyxa betae. The damage in yield and quality of infected crops is significant. The area where the rhizomania disease is spread is continuously increasing. A chemical control of this virus disease is not possible, therefore the only chance to overcome these problems caused by the rhizomania disease is the development of resistant varieties.

Sugar beet hybrids are bred by self-pollinating and cross pollinating techniques. The development and production of these hybrids requires the development of lines, crossing of these lines and the evaluation of the progenies. Evaluation for virus resistance, especially BNYVV, requires large ressources and current methods have poor reproduceability.

The identification of desirable agronomic traits is traditioall done by phenotypic selection.

In Bodenkultur, Vol. 39, 1988, pp. 251-258, for example, a method for selection of rhizomania resistant beet plants is disclosed. Resistant plants are identified by visual observation as well as by ELISA tests. The resistance of beet plants against rhizomania is supposed to be causally related with the presence of a single dominant allele, which, probably, is present only in special lines of a breeding material.

It is well known that the genomic DNA of two individuals in a species, for example, will differ in sequence at many sites. When these differences occur in the recoanition site for a restriction endonuclease, the enzyme will not cleave the DNA molecule at that point. Likewise, a variation may introduce a recognition site where none exists in the other individual, causing the DNA to be cut by the restriction enzyme at that point. Because of this, digestion of the two individuals' DNA will produce fragments having different length. A polymorphism in the length of restriction fragments produced by digestion of the DNA of the two individuals will result. Differences in the lengths cf sequences between recognition sites, as well as deletions, amplifications, translocations, inversions and other events may cause polymorphism between qenotypically different individuals.

It would be valuable to plant breeders to be able to identify aenes affecting agronomic traits on the molecular level through the identification of linked genetic markers. Technology now provides a method for identifying genetic markers with potential application in plant breeding through the use of Restriction Fraament Length Polymorphisms (RFLPs). It is therefore of great importance to investigate the possibility of using genetic linkage analysis between DNA polymorphisms and traits of agronomic importance in order to identify agronomically important aenes, to classify inbreds, hybrids and breeding population according to the structure of their genomes, and thus to effectively incorporate new traits into improved inbreds and hybrids (Tanksley S.D., Young N.D., Paterson A.H., Bonierbale M.W. (1989). "RFLP mapping in plant breeding: new tools for an old science". Bio/Technology 7: 257-264.)

The reference WO 89/07647 is dealing with the use of restriction fragment length polymorphisms identified by use of specific DNA probes to identify genetic linkages with agronomically important genes in corn.

### Problem to be solved by the invention

The problem to be solved by the present invention was to enhance the selection process in beta beet breeding. More precisely the problem to be solved by the present invention was to provide a method which allows plant breeders to identify rhizomania resistant beta beet plants or beet seeds and to distinguish them from rhizomania susceptible beta beet plants or beet seeds safer and faster than by using the conventional methods of beta beet resistance breeding.

### Summary of the invention

The present invention is based on the use of a RFLP probe to identify genetic linkages to an agronomically important trait, comprising genes coding for resistance against rhizomania disease. Specifically the present invention consists of a method for locating genes coding for resistance against rhizomania disease.

The present invention is based on the discovery, that rhizomania resistant beta beet plants and beta beet seeds, for example sugar beet plants and sugar beet seeds, can easily and fast be identified and distinguished from rhizomania susceptible plants and seeds by a method which is characterized therein, that (a) specifically restricted genomic DNA from a beta beet plant or beet seed, which has to be analyzed, is treated with a RFLP probe (Restriction Fragment Length Polymorphism probe), capable of combining with genomic DNA closely linked to genes coding for resistance against rhizomania disease and that (b) any genomic DNA comprising genes coding for resistance against rhizomania disease is marked with said RFLP probe.

Accordingly the present invention is based on the discovery, that a RFLP probe can be used as a molecular marker to identify genetic linkages with genes coding for resistance against rhizomania disease.

The present invention comprises two major parts namely:
- 1.: the use of a RFLP probe (DNA probe) to reveal polymorphisms between breeding material and
- 2.: the use of the identified genetic linkage between a specific RFLP probe and the genetic component of an agronomically important trait in beta beet, particularly sugar beet breeding.

Specifically the present invention is based on the identification of a restriction fragment of a beta beet plant or beta beet seed, especially sugar beet plant or seed, that defines genetic linkage between an agronomically important trait comprising genes coding for resistance against rhizomania disease using analytical techniques that can find correlations between the inheritance of a DNA sequence and the phenotype of the plant under investigation.

Identifying RFLP's involves the use of restriction endonucleases, DNA mapping, and cloned DNA probes. Restriction endonucleases cleave the genomic DNA molecules at specific sites.

Certain polymorphisms can thus be used as genetic markers or RFLP probes, that are associated with genomic DNA comprising genes coding for resistance against rhizomania disease. Establishment of such an association permits the monitoring of heritable sequences of genomic DNA.

This marker or RFLP probe that identifies this genetic characteristic is particularly useful in a breeding programme in order to select for this trait.

In general, to identify a polymorphism according to this invention, DNA is extracted from the plant cell and digested with a given restriction endonuclease. After the digest is obtained, and the same is separated by a standard technique such as, for example, agarose gel electrophoresis, the separated bands are probed with a DNA fragment coding for the RFLP sequence.

A probe must ultimately be found to detect a polymorphism if it is to be useful for testing for the desired trait. The polymorphism must be found to be linked to genes affecting traits.

A population of sugar beet plants is typically found, for example, in the progeny of selfed F₁ hybrids of two different inbred lines. Once the population has been identified, RFLP profiles of each plant are performed using the well-known techniques described above.

A preferred embodiment of the present invention comprises:
(a) digesting genomic DNA from a beta beet plant or a beet seed with restriction endonucleases like Taq I, Alu I, Rsa I that produce a Restriction Fragment Length Polymorphism digestion pattern that is associated with rhizomania disease;
(b) separating the fragments obtained from said digestion in step (a);
(c) detecting said Restriction Fragment Length Polymorphism with RFLP probe containing sequence information capable of hybridizing to and identifying said probe and
(d) identifying individual beta beet plants or beet seeds which have the desired genotype for resistance to rhizomania by correlating the presence or absence of the signal from said probe in said digest with the respective presence or absence of said rhizomania disease,
characterised in that
a RFLP probe is used, which is characterized by the following sequence of bp:

10 30 50
. . . . . .
GAAAAATGAAAATCGCAACTCAGGAATTCATGGTCTTTTGACGTGGAATCTTCAGATTC
---------+---------+---------+---------+---------+---------+
CTTTTTACTTTTAGCGTTGAGTCCTTAAGTACCAGAAAACTGCACCTTAGAAGTCTAAG

70 90 110
. . . . . .
TACCCCCACCAGGACTTCAGCTCTCGGAAGCACATCAAGCAACAGCATCTCAGTGGAAGA
---------+---------+---------+---------+---------+---------+
ATGGGGGTGGTCCTGAAGTCGAGAGCCTTCGTGTAGTTCGTTGTCGTAGAGTCACCTTCT

130 150 170
. . . . . .
TAACAAAGGTCAGGATATAAAAAATGCTTCATTTGCAAGAGTGAAGTTAAGGAAACATTT
---------+---------+---------+---------+---------+---------+
ATTGTTTCCAGTCCTATATTTTTTACGAAGTAAACGTTCTCACTTCAATTCCTTTGTAAA

190 210 230
. . . . . .
GGCTTTTGACAGATCAAAAAGCGAAAGCCAAATGGAAGTTGATGACTCCCCAAATTGTCA
---------+---------+---------+---------+---------+---------+
CCGAAAACTGTCTAGTTTTTCGCTTTCGGTTTACCTTCAACTACTGAGGGGTTTAACAGT

250 270 290
. . . . . .
CAATACACTAGCTCAAATGATAGCAATTCAATCCAATATGCAACAAGAAAACAGAGAAAT
---------+---------+---------+---------+---------+---------+
GTTATGTGATCGAGTTTACTATCGTTAAGTTAGGTTATACGTTGTTCTTTTGTCTCTTTA

310 330 350
. . . . . .
GAAAGAGAAGCTAGATAGAGTAACTGAAGAGAATGTAGCACTGATGAACCAACTTGAAGA
---------+---------+---------+---------+---------+---------+
CTTTCTCTTCGATCTATCTCATTGACTTCTCTTACATCGTGACTACTTGGTTGAACTTCT

370 390 410
. . . . . .
ATCAAAGCAGACCATTGAGAGCTTACAAAGGGAGCTAGAATCTCTCAAGGAAGCAAATGG
---------+---------+---------+---------+---------+---------+
TAGTTTCGTCTGGTAACTCTCGAATGTTTCCCTCGATCTTAGAGAGTTCCTTCGTTTACC

430 450 470
. . . . . .
GAATATTGAGGAACAAGTTGAAAATCAGAGGTTAATAAATGAAGATCTTGATACACAGTA
---------+---------+---------+---------+---------+---------+
CTTATAACTCCTTGTTCAACTTTTAGTCTCCAATTATTTACTTCTAGAACTATGTGTCAT

490 510 530
. . . . . .
TACAGTTGCAAAAGCCAAGCTAAATGAAATTCTCCATAAATTATCAACTTTAGAGGTAGA
---------+---------+---------+---------+---------+---------+
ATGTCAACGTTTTCGGTTCGATTTACTTTAAGAGGTATTTAATAGTTGAAATCTCCATCT

550 570 590
. . . . . .
ATTGGAGGATAAGAATAACTGCTGTGAAGAATTAGAAGCAACATGCCTTGAACTTCAGCT
---------+---------+---------+---------+---------+---------+
TAACCTCCTATTCTTATTGACGACACTTCTTAATCTTCGTTGTACGGAACTTGAAGTCGA

610 630 650
. . . . . .
TCAGCTTGAAACGTAAGAAGATATAATCCTTTTGCAGGTTCATTTAACTTCTTAGAAACT
---------+---------+---------+---------+---------+---------+
AGTCGAACTTTGCATTCTTCTATATTAGGAAAACGTCCAAGTAAATTGAAGAATCTTTGA

670 690 710
. . . . . .
AGTGATAGTGCACACTCATGCTCACACACAGGTACGCTTGCATGGATAATCTAATCTATG
---------+---------+---------+---------+---------+---------+
TCACTATCACGTGTGAGTACGAGTGTGTGTCCATGCGAACGTACCTATTAGATTAGATAC

TGCCTG
------
ACGGAC

### Development of a RFLP probe

Said RFLP probe, which is used in a highly preferred embodiment of the present invention was developed as follows:

In 1988 two sugar beet hybrids (F₁) using the following lines were produced:
- R01 (monogerm, resistant to rhizomania)
- N01 (monogerm, susceptible to rhizomania)
- N02 (monogerm, susceptible to rhizomania)
- R02 (multigerm, resistant to rhizomania)

The two hybrids were crossed as follows:
- R01 x N01
- R02 x N02

In 1989 both hybrids were crossed with each other as follows:
A: (R01 x N01) x (R02 x N02) and of each hybrid S₁-lines where produced by selfing (SE):
B: SE (R01 x N01)
C: SE (R02 x N02)

In 1990 out of the 4-way-cross A a segregating population was produced which consisted of 49 individuals. All 49 plants were selfed on the one hand and at the same time they were micropropagated and stored in vitro. Out of the S₁-lines of cross B (R01 x N01) and cross C (R02 x N02) S₂-lines were produced by selfing.

### Sugar beet DNA Isolation

Total DNA can be isolated from various tissues (leaves, seedlings, etc.) by any one of several standard methods (for example see Maniatis et al., Molecular Cloning, A Laboratory Manual (1982); Dillon et al., Recombinat DNA Methodology (John Wiley & Sons 1985).

### Identification of Informative DNA Probes (Landry B.S., Michel-R.W. (1985. "Selection of probes for restriction fragment length analysis from plant genomic clones". Plant Mol. Biol. Rep. 3: 174-179).

Total DNA was purified and digested with the restriction enzyme PstI. After size fractionation on a sucrose gradient fragments from the class between 500 and 2.000 bp were ligated into the PstI digested Bluescript vector. Transformation into the E.coli strain TG-2 was done using standard methods. Clones giving no hybridization signal with ³²P-labelled total sugar beet DNA were used as markers. Recombinant plasmids were purified according to Birnboim and Doly (1979), "A rapid alkaline extraction procedure for screening recombinant plasmid DNA." Nucleic Acids Res. 7: 1513-1523. Inserts were isolated by elution from low melting agarose gels and labelled with ³²P-α-dCTP (Amersham) using the random primer labelling method of Feinberg and Vogelstein (1983), Feinberg AP, Vogelstein B (1983) "A technique for radiolabelling DNA restriction endonuclease fragments to high specific activity." Anal Biochem 132:6-13 and of Feinberg AP, Vogelstein B (1984) "A technique for radiolabelling DNA restriction endonuclease fragments to high specific activity". (Addendum). Anal Biochem 137:266-267.

Southern hybridization was done using a labelled sugar beet insert as probe and total DNA from various sugar beet plants digested to completion with the restriction enzymes AluI, RsaI and TaqI as target. After washing the membrane to remove any nonhybridized probe, the membranes were subjected to autoradiography (Gebhardt et al. 1989) "RFLP analysis and linkage mapping in Solanum tuberosum." Theor. Appl. Genet. 78: 65-75.

Probes were selected for further consideration if (1) one autoradiographic band was observed for at least one restriction enzyme, and (2) band patterns were different between at least two sugar beet plants.

### Determination of the Correlation between Phenotypic Expression of the Trait and a Specific Probe (Ritter et al. (1990) "Estimation of recombination frequencies and construction of RFLP linkage maps in plants from crosses between heterozygous parents." Genetics 125:645-654)

In the winter of 1990/91 a rhizomania-resistance-test was done in the glasshouse. The seedlings of all inbreads of A, B and C were tested in rhizomania-infested soil.

Seedling plants were assayed for virus concentration by standard Elisa techniques. Plants were classified into 2 classes, "resistant" and "susceptible".

Results of the 2 classes were compared with a set of RFLP-probes. One probe recognized plants classified as resistant. These results were confirmed in follow-up test, performed on breeding material with various genetic background.

## Claims

1. A method for identifying rhizomania resistant beta beet plants or beet seeds and distinguishing them from rhizomania susceptible beta beet plants or beet seed, wherein
(a) specifically restricted genomic DNA from a beta beet plant or a beet seed, which has to be analyzed, is treated with a RFLP probe (Restriction Fragment Length Polymorphism probe), capable of combining with genomic DNA closely linked to genes coding for resistance against rhizomania disease, and
(b) any genomic DNA comprising genes coding for resistance against rhizomania disease is marked with said RFLP probe, characterized in that said RFLP probe comprises the following sequence of basepairs:

10        30        50
.         .         .         .         .         .
GAAAAATGAAAATCGCAACTCAGGAATTCATGGTCTTTTGACGTGGGAATCTTCAGATTC
---------+---------+---------+---------+---------+---------+
CTTTTTACTTTTAGCGTTGAGTCCTTAAGTACCAGAAAACTGCACCCTTAGAAGTCTAAG

70        90        110
.         .         .         .         .         .
TACCCCCACCAGGACTTCAGCTCTCGGAAGCACATCAAGCAACAGCATCTCAGTGGAAGA
---------+---------+---------+---------+---------+---------+
ATGGGGGTGGTCCTGAAGTCGAGAGCCTTCGTGTAGTTCGTTGTCGTAGAGTCACCTTCT

130       150       170
.         .         .         .         .         .
TAACAAAGGTCAGGATATAAAAAATGCTTCATTTGCAAGAGTGAAGTTAAGGAAACATTT
---------+---------+---------+---------+---------+---------+
ATTGTTTCCAGTCCTATATTTTTTACGAAGTAAACGTTCTCACTTCAATTCCTTTGTAAA

190       210       230
.         .         .         .         .         .
GGCTTTTGACAGATCAAAAAGCGAAAGCCAAATGGAAGTTGATGACTCCCCAAATTGTCA
---------+---------+---------+---------+---------+---------+
CCGAAAACTGTCTAGTTTTTCGCTTTCGGTTTACCTTCAACTACTGAGGGGTTTAACAGT

250 270 290
. . . . . .
CAATACACTAGCTCAAATGATAGCAATTCAATCCAATATGCAACAAGAAAACAGAGAAAT
---------+---------+---------+---------+---------+---------+
GTTATGTGATCGAGTTTACTATCGTTAAGTTAGGTTATACGTTGTTCTTTTGTCTCTTTA

310 330 350
. . . . . .
GAAAGAGAAGCTAGATAGAGTAACTGAAGAGAATGTAGCACTGATGAACCAACTTGAAGA
---------+---------+---------+---------+---------+---------+
CTTTCTCTTCGATCTATCTCATTGACTTCTCTTACATCGTGACTACTTGGTTGAACTTCT

370 390 410
. . . . . .
ATCAAAGCAGACCATTGAGAGCTTACAAAGGGAGCTAGAATCTCTCAAGGAAGCAAATGG
---------+---------+---------+---------+---------+---------+
TAGTTTCGTCTGGTAACTCTCGAATGTTTCCCTCGATCTTAGAGAGTTCCTTCGTTTACC

430 450 470
. . . . . .
GAATATTGAGGAACAAGTTGAAAATCAGAGGTTAATAAATGAAGATCTTGATACACAGTA
---------+---------+---------+---------+---------+---------+
CTTATAACTCCTTGTTCAACTTTTAGTCTCCAATTATTTACTTCTAGAACTATGTGTCAT

490 510 530
. . . . . .
TACAGTTGCAAAAGCCAAGCTAAATGAAATTCTCCATAAATTATCAACTTTAGAGGTAGA
---------+---------+---------+---------+---------+---------+
ATGTCAACGTTTTCGGTTCGATTTACTTTAAGAGGTATTTAATAGTTGAAATCTCCATCT

550 570 590
. . . . . .
ATTGGAGGATAAGAATAACTGCTGTGAAGAATTAGAAGCAACATGCCTTGAACTTCAGCT
---------+---------+---------+---------+---------+---------+
TAACCTCCTATTCTTATTGACGACACTTCTTAATCTTCGTTGTACGGAACTTGAAGTCGA

610 630 650
. . . . . .
TCAGCTTGAAACGTAAGAAGATATAATCCTTTTGCAGGTTCATTTAACTTCTTAGAAACT
---------+---------+---------+---------+---------+---------+
AGTCGAACTTTGCATTCTTCTATATTAGGAAAACGTCCAAGTAAATTGAAGAATCTTTGA

670 690 710
. . . . . .
AGTGATAGTGCACACTCATGCTCACACACAGGTACGCTTGCATGGATAATCTAATCTATG
---------+---------+---------+---------+---------+---------+
TCACTATCACGTGTGAGTACGAGTGTGTGTCCATGCGAACGTACCTATTAGATTAGATAC

TGCCTG
------
ACGGAC

2. The method according to claim 1, comprising:
(a) digesting genomic DNA from a beta beet plant or a beet seed with restriction endonucleases like TaqI, AluI and RsaI that produce a Restriction Fragment Length Polymorphism digestion pattern that is associated with rhizomania disease;
(b) separating the fragments obtained from said digestion in step (a);
(c) detecting said Restriction Fragment Length Polymorphism with a RFLP probe comprising the sequence of basepairs according to Claim 1, and
(d) identifying individual beta beet plants or beet seeds which have the desired genotype for resistance to rhizomania by correlating the presence or absence of the signal from said probe in said digest with the respective presence or absence of said rhizomania disease.

3. A RFLP probe for the identification of rhizomania resistant beta beet plants, or beet seeds characterized by a DNA sequence in basepairs homologous to the one shown in Claim 1.

4. DNA constructs (double-stranded cDNAs) able to identify rhizomania resistant beta beet plants or beet seeds and to distinguish them from rhizomania susceptible beta beet plants or beet seeds comprising one or more sequences of base pair combinations being strictly homologous to sequence portions within the RFLP probe as described in Claim 3 or comprising modifications within said sequences which still allow for hybridizing to said RFLP probe.

5. A method for selecting for rhizomania resistant beet plants or seeds comprising use of the RFLP probe as described in any of Claims 1 to 3 in combination with restriction enzymes like TaqI, AluI and RsaI or any other suitable restriction enzyme or combination of restriction enzymes.

## Patentansprüche

1. Verfahren zur Identifizierung von Rizomania- bzw. Wundbärtigkeit-resistenten Beta-Rübenpflanzen oder-Rübensaatgutkeimen und zur Unterscheidung dieser von Rizomaniaempfindlichen Beta-Rübenpflanzen oder -Rübensaatgutkeimen, wobei
(a) in spezifischer Weise restriktionsverdaute genomische DNA von einer Beta-Rübenpflanze oder einem -Rübensaatgutkeim, welche(r) zu analysieren ist, mit einer RFLP-Sonde (Restriktionsfragment-Längenpolymorphismus-Sonde), die in der Lage ist, an genomischer DNA, welche eng mit für Resistenz gegen die Rizomania-Erkrankung codierenden Genen gekoppelt ist, zu binden, behandelt wird, und
(b) jedwede genomische DNA, die für Resistenz gegen die Rizomania-Erkrankung codierende Gene umfaßt, mit dieser RFLP-Sonde markiert wird, dadurch gekennzeichnet daß die RFLP-Sonde die folgende Basenpaarsequenz beinhaltet:

10          30          50
.     .     .     .     .     .
GAAAAATGAAAATCGCAACTCAGGAATTCATGGTCTTTTGACGTGGGAATCTTCAGATTC
---------+---------+---------+---------+---------+---------+
CTTTTTACTTTTAGCGTTGAGTCCTTAAGTACCAGAAAACTGCACCCTTAGAAGTCTAAG

70          90          110
.     .     .     .     .     .
TACCCCCACCAGGACTTCAGCTCTCGGAAGCACATCAAGCAACAGCATCTCAGTGGAAGA
---------+---------+---------+---------+---------+---------+
ATGGGGGTGGTCCTGAAGTCGAGAGCCTTCGTGTAGTTCGTTGTCGTAGAGTCACCTTCT

130          150          170
.     .     .     .     .     .
TAACAAAGGTCAGGATATAAAAAATGCTTCATTTGCAAGAGTGAAGTTAAGGAAACATTT
---------+---------+---------+---------+---------+---------+
ATTGTTTCCAGTCCTATATTTTTTACGAAGTAAACGTTCTCACTTCAATTCCTTTGTAAA

190          210          230
.     .     .     .     .     .
GGCTTTTGACAGATCAAAAAGCGAAAGCCAAATGGAAGTTGATGACTCCCCAAATTGTCA
---------+---------+---------+---------+---------+---------+
CCGAAAACTGTCTAGTTTTTCGCTTTCGGTTTACCTTCAACTACTGAGGGGTTTAACAGT

250                 270                 290
    .         .         .         .         .         .
CAATACACTAGCTCAAATGATAGCAATTCAATCCAATATGCAACAAGAAAACAGAGAAAT
---------+---------+---------+---------+---------+---------+
GTTATGTGATCGAGTTTACTATCGTTAAGTTAGGTTATACGTTGTTCTTTTGTCTCTTTA

310                 330                 350
    .         .         .         .         .         .
GAAAGAGAAGCTAGATAGAGTAACTGAAGAGAATGTAGCACTGATGAACCAACTTGAAGA
---------+---------+---------+---------+---------+---------+
CTTTCTCTTCGATCTATCTCATTGACTTCTCTTACATCGTGACTACTTGGTTGAACTTCT

370                 390                 410
    .         .         .         .         .         .
ATCAAAGCAGACCATTGAGAGCTTACAAAGGGAGCTAGAATCTCTCAAGGAAGCAAATGG
---------+---------+---------+---------+---------+---------+
TAGTTTCGTCTGGTAACTCTCGAATGTTTCCCTCGATCTTAGAGAGTTCCTTCGTTTACC

430                 450                 470
    .         .         .         .         .         .
GAATATTGAGGAACAAGTTGAAAATCAGAGGTTAATAAATGAAGATCTTGATACACAGTA
---------+---------+---------+---------+---------+---------+
CTTATAACTCCTTGTTCAACTTTTAGTCTCCAATTATTTACTTCTAGAACTATGTGTCAT

490                 510                 530
    .         .         .         .         .         .
TACAGTTGCAAAAGCCAAGCTAAATGAAATTCTCCATAAATTATCAACTTTAGAGGTAGA
---------+---------+---------+---------+---------+---------+
ATGTCAACGTTTTCGGTTCGATTTACTTTAAGAGGTATTTAATAGTTGAAATCTCCATCT

550                 570                 590
    .         .         .         .         .         .
ATTGGAGGATAAGAATAACTGCTGTGAAGAATTAGAAGCAACATGCCTTGAACTTCAGCT
---------+---------+---------+---------+---------+---------+
TAACCTCCTATTCTTATTGACGACACTTCTTAATCTTCGTTGTACGGAACTTGAAGTCGA

610                 630                 650
    .         .         .         .         .         .
TCAGCTTGAAACGTAAGAAGATATAATCCTTTTGCAGGTTCATTTAACTTCTTAGAAACT
---------+---------+---------+---------+---------+---------+
AGTCGAACTTTGCATTCTTCTATATTAGGAAAACGTCCAAGTAAATTGAAGAATCTTTGA

670                 690                 710
    .         .         .         .         .         .
AGTGATAGTGCACACTCATGCTCACACACAGGTACGCTTGCATGGATAATCTAATCTATG
---------+---------+---------+---------+---------+---------+
TCACTATCACGTGTGAGTACGAGTGTGTGTCCATGCGAACGTACCTATTAGATTAGATAC

TGCCTG
------
ACGGAC

2. Verfahren nach Anspruch 1, umfassend:
(a) Verdauen genomischer DNA von einer Beta-Rübenpflanze oder einem -Rübensaatgutkeim mit Restflktionsendonukleasen wie TaqI, AluI und RsaI, welche ein Restriktionsfragment-Längenpolymorphismus-Verdauungsmuster erzeugen, das mit der Rizomania-Erkrankung in Verbindung steht;
(b) Trennen der aus dem Verdau in Schritt (a) erhaltenen Fragmente;
(c) Detektieren des Restriktionsfragment-Längenpolymorphismus mit einer RFLP-Sonde, umfassend die Basenpaarsequenz gemäß Anspruch 1; und
(d) Identifizieren einzelner Beta-Rübenpflanzen oder -Rübensaatgutkeime, welche den gewünschten Genotyp für die Resistenz gegenüber Rizomania aufweisen, indem die Gegenwart oder Abwesenheit des Signals der Sonde in dem Verdau mit der entsprechenden Gegenwart oder Abwesenheit der Rizomania-Erkrankung korreliert wird.

3. RFLP-Sonde für die Identifizierung von Rizomania-resistenten Beta-Rübenpflanzen oder -Rübensaatgutkeimen, gekennzeichnet durch eine DNA-Sequenz, die bezüglich der Basenpaare homolog zu der in Anspruch 1 gezeigten ist.

4. DNA-Konstrukte (doppelsträngige cDNAs), die in der Lage sind, Rizomania-resistente Beta-Rübenpflanzen oder -Rübensaatgutkeime zu identifizieren und diese von Rizomania-empfindlichen Beta-Rübenpflanzen oder -Rübensaatgutkeimen zu unterscheiden, umfassend eine oder mehrere Sequenzen von Basenpaarkombinationen, welche streng homolog zu Sequenzabschnitten innerhalb der RFLP-Sonde, wie beschrieben in Anspruch 3, sind oder Modifikationen innerhalb dieser Sequenzen umfassen, welche immer noch eine Hybridisierung mit der RFLP-Sonde ermöglichen.

5. Verfahren zur Auswahl bezüglich Rizomania-resistenten Beta-Rübenpflanzen oder -Rübensaatgutkeimen, umfassend die Verwendung der RFLP-Sonde, wie sie in einem beliebigen der Ansprüche 1 bis 3 beschrieben ist, in Kombination mit Restriktionsenzymen wie TaqI, AluI und RsaI oder jedwedem anderen geeigneten Restriktionsenzym oder einer Kombination von Restriktionsenzymen.

## Revendications

1. Procédé pour identifier des plants de betterave ou des graines de betterave résistants à la rhizomanie de la betterave, et pour les distinguer des plants de betterave ou de graines de betterave sensibles à la rhizomanie, dans lequel
(a) on traite un ADN génomique d'un plant ou d'une graine de betterave à analyser, coupé de façon spécifique par des enzymes de restriction, avec une sonde de RFLP (sonde de polymorphisme de la longueur des fragments de restriction) capable de se combiner avec un ADN génomique étroitement lié à des gènes codant pour la résistance à la rhizomanie de la betterave, et
(b) on marque avec ladite sonde de RFLP tout ADN génomique comprenant des gènes codant pour la résistance à la rhizomanie de la betterave,
caractérisé en ce que ladite sonde de RFLP comprend la séquence de paires de bases suivante:

10                  30                  50
         .         .         .         .         .         .
GAAAAATGAAAATCGCAACTCAGGAATTCATGGTCTTTTGACGTGGGAATCTTCAGATTC
---------+---------+---------+---------+---------+---------+
CTTTTTACTTTTAGCGTTGAGTCCTTAAGTACCAGAAAACTGCACCCTTAGAAGTCTAAG

        70                  90                 110
         .         .         .         .         .         .
TACCCCCACCAGGACTTCAGCTCTCGGAAGCACATCAAGCAACAGCATCTCAGTGGAAGA
---------+---------+---------+---------+---------+---------+
ATGGGGGTGGTCCTGAAGTCGAGAGCCTTCGTGTAGTTCGTTGTCGTAGAGTCACCTTCT

       130                 150                 170
         .         .         .         .         .         .
TAACAAAGGTCAGGATATAAAAAATGCTTCATTTGCAAGAGTGAAGTTAAGGAAACATTT
---------+---------+---------+---------+---------+---------+
ATTGTTTCCAGTCCTATATTTTTTACGAAGTAAACGTTCTCACTTCAATTCCTTTGTAAA

       190                 210                 230
         .         .         .         .         .         .
GGCTTTTGACAGATCAAAAAGCGAAAGCCAAATGGAAGTTGATGACTCCCCAAATTGTCA
---------+---------+---------+---------+---------+---------+
CCGAAAACTGTCTAGTTTTTCGCTTTCGGTTTACCTTCAACTACTGAGGGGTTTAACAGT

       250                 270                 290
         .         .         .         .         .         .
CAATACACTAGCTCAAATGATAGCAATTCAATCCAATATGCAACAAGAAAACAGAGAAAT
---------+---------+---------+---------+---------+---------+
GTTATGTGATCGAGTTTACTATCGTTAAGTTAGGTTATACGTTGTTCTTTTGTCTCTTTA

310 330 350
. . . . . .
GAAAGAGAAGCTAGATAGAGTAACTGAAGAGAATGTAGCACTGATGAACCAACTTGAAGA
---------+---------+---------+---------+---------+---------+
CTTTCTCTTCGATCTATCTCATTGACTTCTCTTACATCGTGACTACTTGGTTGAACTTCT

370 390 410
. . . . . .
ATCAAAGCAGACCATTGAGAGCTTACAAAGGAGCTAGAATCTCTCAAGGAAGCAAATGG
---------+---------+---------+---------+---------+---------+
TAGTTTCGTCTGGTAACTCTCGAATGTTTCCTCGATCTTAGAGAGTTCCTTCGTTTACC

430 450 470
. . . . . .
GAATATTGAGGAACAAGTTGAAAATCAGAGGTTAATAAATGAAGATCTTGATACACAGTA
---------+---------+---------+---------+---------+---------+
CTTATAACTCCTTGTTCAACTTTTAGTCTCCAATTATTTACTTCTAGAACTATGTGTCAT

490 510 530
. . . . . .
TACAGTTGCAAAAGCCAAGCTAAATGAAATTCTCCATAAATTATCAACTTTAGAGGTAGA
---------+---------+---------+---------+---------+---------+
ATGTCAACGTTTTCGGTTCGATTTACTTTAAGAGGTATTTAATAGTTGAAATCTCCATCT

550 570 590
. . . . . .
ATTGGAGGATAAGAATAACTGCTGTGAAGAATTAGAAGCAACATGCCTTGAACTTCAGCT
---------+---------+---------+---------+---------+---------+
TAACCTCCTATTCTTATTGACGACACTTCTTAATCTTCGTTGTACGGAACTTGAAGTCGA

610 630 650
. . . . . .
TCAGCTTGAAACGTAAGAAGATATAATCCTTTTGCAGGTTCATTTAACTTCTTAGAAACT
---------+---------+---------+---------+---------+---------+
AGTCGAACTTTGCATTCTTCTATATTAGGAAAACGTCCAAGTAAATTGAAGAATCTTTGA

670 690 710
. . . . . .
AGTGATAGTGCACACTCATGCTCACACACAGGTACGCTTGCATGGATAATCTAATCTATG
---------+---------+---------+---------+---------+---------+
TCACTATCACGTGTGAGTACGAGTGTGTGTCCATGCGAACGTACCTATTAGATTAGATAC

TGCCTG
------
ACGGAC

2. Procédé selon la revendication 1, comprenant
(a) la digestion d'ADN génomique d'un plant de betterave ou d'une graine de betterave avec des endonucléases de restriction comme *Taq* I, *Alu* I et *Rsa* I, qui produisent un diagramme de digestion à polymorphisme de longueur des fragments de restriction associé à la rhizomanie de la betterave;
(b) la séparation des fragments obtenus par ladite digestion de l'étape (a);
(c) la détection dudit polymorphisme de longueur des fragments de restriction avec la sonde de RFLP comprenant la séquence de paires de bases selon la revendication 1, et
(d) l'identification des plants ou des graines de betterave individuels ayant le génotype désiré pour la résistance à la rhizomanie par la mise en corrélation de la présence ou de l'absence du signal de ladite sonde dans ledit produit de digestion avec, respectivement, la présence ou l'absence de ladite rhizomanie.

3. Sonde de FLRP pour l'identification de plants de betterave ou de graines de betterave résistants à la rhizomanie, caractérisée par une séquence d'ADN en paires de bases homologue à celle indiquée dans la revendication 1.

4. Assemblages d'ADN (ADNc double brin) capables d'identifier les plants de betterave ou les graines de betterave résistants à la rhizomanie et de les distinguer des plants de betterave ou des graines de betterave sensibles à la rhizomanie, comprenant une ou plusieurs séquences de combinaisons de paires de bases strictement homologues à des portions de séquences contenues dans la sonde de RFLP décrite dans la revendication 3 ou comprenant des modifications dans lesdites séquences, qui permettent encore l'hybridation à ladite sonde de RFLP.

5. Procédé de sélection de plants ou de graines de betterave résistants à la rhizomanie, comprenant l'utilisation de la sonde de RFLP selon l'une quelconque des revendications 1 à 3 en combinaison avec des enzymes de restriction comme *Taq* I, *Alu* I et *Rsa* I ou toute autre enzyme de restriction ou combinaison d'enzymes de restriction appropriée.
